(19) Europäisches Patentamt
European Patent Office
Office européen des brevets

(11) Veröffentlichungsnummer: **0 057 893**
B1

(12) **EUROPÄISCHE PATENTSCHRIFT**

(45) Veröffentlichungstag der Patentschrift: **30.11.83**

(51) Int. Cl.³: **C 07 C 51/41,** C 07 C 53/128, C 07 F 3/02

(21) Anmeldenummer: **82100700.2**

(22) Anmeldetag: **02.02.82**

(54) **Organische Barium-Magnesium-Komplexe und Verfahren zu ihrer Herstellung.**

(30) Priorität: **11.02.81 DE 3104791**

(43) Veröffentlichungstag der Anmeldung: **18.08.82 Patentblatt 82/33**

(45) Bekanntmachung des Hinweises auf die Patenterteilung: **30.11.83 Patentblatt 83/48**

(84) Benannte Vertragsstaaten: **BE DE FR GB**

(56) Entgegenhaltungen:
**DE - A - 2 943 357**
**FR - A - 2 433 530**
**GB - A - 1 140 066**
**US - A - 3 846 385**
**US - A - 3 903 019**

(73) Patentinhaber: **SCHERING AKTIENGESELLSCHAFT Berlin und Bergkamen, Waldstrasse 14 Postfach 1540, D-4619 Bergkamen (DE)**

(72) Erfinder: **Müller, Karl, Heinz, Dipl.-Chem. Dr., Grevinghof 55, D-4712 Werne (DE)**
Erfinder: **Schröer, Ulrich, Dipl.-Chem., Dr., Meckeweg 4, D-4618 Kamen-Methler (DE)**

**EP 0 057 893 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Organische Barium-Magnesium-Komplexe und Verfahren zu ihrer Herstellung

Organobariumverbindungen sind nur in wenigen Spezialfällen sicher bekannt, so z. B. Dimethyl- und Diphenylbarium. Ihre Haltbarkeit ist begrenzt. Komplexe von Ba-alkylen oder -alkylhydriden mit Zn-, Al- und B-Verbindungen sind bekannt und haben weitgehend Salzcharakter.

In der US-PS 3 903 019 sind in Kohlenwasserstoffen lösliche Butyl-sec.-butyl-magnesium-Komplexe mit Barium-tert.-butylat beschrieben. Die Komplexe sind jedoch aufgrund der aufwendigen Herstellung der Bariumalkoxide, welche durch Umsetzung von metallischem Barium mit tert. Alkohol in Aminen als Lösungsmittel unter Inertgas nach Abtrennen des überschüssigen Alkohols und der Amine erhalten werden, entsprechend schwierig und aufwendig zu erhalten.

Es wurde nun gefunden, daß man in ätherfreien Kohlenwasserstoffen lösliche organische Barium-Magnesium-Komplexe in einfacher Weise und in guten Ausbeuten erhält, wenn man Bariumcarboxylate mit Magnesiumdialkylen umsetzt.

(Die erhaltenen Verbindungen sind wahrscheinlich assoziiert; der Einfachheit halber sind sie hier und im folgenden als Monomere dargestellt. Die in Formel I gewählte Darstellungsweise wurde, ausgehend von den eingesetzten Verbindungen, der Übersichtlichkeit halber gewählt und soll nicht die tatsächlichen Bindungsverhältnisse und Strukturen wiedergegeben.)

Gegenstand der Erfindung ist daher ein Verfahren zur Herstellung von in Kohlenwasserstoffen löslichen Barium-Magnesium-Alkylkomplexverbindungen der Formel

$$[Mg\,R^1_aR^2_b]_x\left[Ba\left(O\underset{\underset{O}{\|}}{C}-R^3\right)_2\right]_y \qquad (1)$$

in der $R^1$ und $R^2$ gleiche oder verschiedene geradkettige oder verzweigte Alkylreste, vorzugsweise mit 1–8 C-Atomen, oder Aryl- oder Aralkylreste bedeuten, in der $R^3$ ein Alkylrest mit mindestens 2 C-Atomen, insbesondere ein Alkylrest mit mehr als 5 C-Atomen, ist, wobei (a+b) gleich 2 und das Verhältnis x zu y 1 bis 4 beträgt, das dadurch gekennzeichnet ist, daß man Diorganomagnesiumverbindungen der Formel

$$R^1_aR^2_b\,Mg \qquad (2)$$

mit Verbindungen der Formel

$$Ba\left(O\underset{\underset{O}{\|}}{C}-R^3\right)_2$$

bei Temperaturen von 20–130°C, vorzugsweise bei 80–120°C, im Molverhältnis 1 : 1 bis 4 : 1 in Kohlenwasserstoffen umsetzt.

Ein weiterer Gegenstand der Erfindung sind die Verbindungen der allgemeinen Formel

$$[Mg\,R^1_aR^2_b]_x\left[Ba\left(O\underset{\underset{O}{\|}}{C}-R^3\right)_2\right]_y$$

bevorzugt solche, in der $R^1$ Butyl und $R^2$ Octyl bedeutet, insbesondere solche mit einem Butyl-Octyl-Verhältnis von 3 : 1.

Ferner bevorzugt sind Verbindungen, bei denen $R^3$ ein Neononyl-Rest ist, insbesondere die Verbindung

$$Ba\,Mg\,Bu_{1,5}Oc_{0,5}\left(O\underset{\underset{O}{\|}}{C}-\text{neo-Nonyl}\right)_2 \qquad (3)$$

Gegenstand der Erfindung sind ferner stabile Lösungen der genannten Verbindungen in inerten Kohlenwasserstoffen.

Bei Einsatz der niederen Bariumacylate ($R^3 = C_3$ bis $C_8$) ist ein Überschuß an Diorganomagnesiumverbindung erforderlich, beim Bariumpropionat wird beispielsweise zur vollständigen Auflösung die Diorganomagnesiumverbindung im vierfachen Überschuß eingesetzt. Größere Überschüsse an Diorganomagnesium-Verbindungen können bei Bedarf ohne nachteilige Folgen eingesetzt werden.

Diorganomagnesium-Verbindungen sind handelsübliche Verbindungen, die als solche eingesetzt

werden können. Besonders geeignet sind die in Kohlenwasserstoffen lösliche Dialkylmagnesium-Verbindungen, beispielsweise solche mit verzweigten Alkylresten, wie Di-sec.-butylmagnesium oder Butyl-sec.-butylmagnesium. Weiterhin können die Dialkylmagnesium-Verbindungen eingesetzt werden, die Äther oder andere Komplexbildner wie Aluminiumalkyle zu 1 bis 10 Mol-% enthalten. Derartige Magnesiumdialkyle können beispielsweise gemäß US-PS 3 755 478, DE-PS 2 027 327 oder GB-PS 955 806 hergestellt werden.

Auch die einzusetzenden Bariumcarboxylate sind handelsübliche, unempfindliche und leicht herstellbare Verbindungen.

Die erfindungsgemäß hergestellten Komplexe sind somit gut zugänglich und erheblich leichter herstellbar als die oben genannten Barium-tert.-alkoholat-Komplexe gemäß US-PS 3 903 019.

Die Ausgangsverbindungen können als Einzelverbindungen, als Gemische der Einzelverbindungen oder als gemischte Diorganomagnesium-Verbindungen bzw. Bariumdiacylatverbindungen eingesetzt werden.

Besonders eignen sich Bariumcarboxylate mit längerkettigen Säureresten mit mehr als 5 C-Atomen. Günstig wirken sich Kettenverzweigungen auf Umsetzungsgeschwindigkeit und Ausbeute aus. Als besonders geeignet hat sich das Bariumneodecanoat erwiesen.

Die an sich festen Bariumdiacylate lösen sich bei erfindungsgemäßer Umsetzung mit den Diorganomagnesium-Verbindungen rasch auf, wobei bei reinen Ausgangsverbindungen kein Rückstand verbleibt. Es ergeben sich klare, gelbe bis rötliche Lösungen der Endprodukte, die niedrigviskos sind.

Die nach Abtrennung des Lösungsmittels erhaltenen Konzentrate haben höhere Viskositäten und können sogar wachsartig sein, je nach Organo- und Diacylrest.

Weder die Lösungen noch die Konzentrate zeigen nach 6wöchigem Stehen eine Kristallisationsneigung.

Die erfindungsgemäß hergestellten Produkte sind löslich in inerten polaren und unpolaren Lösungsmitteln und können somit auch in ätherfreien Kohlenwasserstoffen eingesetzt werden.

Die erfindungsgemäß hergestellten Verbindungen können als Katalysatoren bzw. Katalysatorkomponente für die Polymerisation oder Copolymerisation genutzt werden.

Derartige Katalysatoren sind beispielsweise geeignet für die Polymerisation von Vinyl- und Allylverbindungen, Lactonen, Lactamen, $\alpha$-Olefinen, besonders Äthylen und Propylen, Dienen, besonders Butadien und Isopren, Epoxiden, Aldehyden, Pyrrolderivaten und anderen.

Für sich oder je nach Art und Menge zusätzlicher Katalysatorkomponenten können Katalysatoren mit hoher Stereospezifität und Wirksamkeit hergestellt werden. Besonders geeignet sind sie für die Copolymerisation von Dienen.

## Beispiele

Wegen der Luftempfindlichkeit der Organomagnesium-Verbindungen und der Endprodukte sind die Reaktionen unter Inertgas durchzuführen.

## Vergleichsbeispiel

Zu 0,1 Mol wasserfreiem Bariumacetat wurde eine Lösung von 0,12 Mol $(C_4H_9)_{1,5}(C_8H_{17})_{0,5}$ Mg in Isopar® E (Isopar® ist ein Gemisch von verschieden verzweigten $C_{10}$-, $C_{11}$- und $C_{12}$-Isoparaffinen) gegeben; diese Lösung enthielt 2,56 Gew.-% Mg und 0,07 Gew.-% Al.

Es trat keine Wärmetönung auf. Die Mischung wurde unter Rühren 4 h auf 115° C erhitzt und danach durch Zentrifugieren geklärt. Die klare, organische Phase wurde mit verdünnter HCl zersetzt und die wäßrige Phase für Barium-frei gefunden.

## Beispiel 1

$$BaMgBu_{1,5}Oc_{0,5}(O_2C-C_9H_{19})_2$$

0,2 Mol Bariumneodecanoat (95,87 g — hergestellt durch azeotropes Entfernen des Reaktionswassers aus Bariumhydroxid und einem Säuregemisch von isomeren Neodecansäuren) wurden vorgelegt und unter Stickstoff mit 0,2 Mol $Bu_{1,5}Oc_{0,5}$ Mg als 15%ige Lösung in Isopar® E versetzt. Die Lösung enthielt 2,19 Gew.-% Mg neben 0,05 Gew.-% Al.

Unter Rühren wurde 1 h auf 60—80°C erwärmt, wobei sich eine rotbraune Lösung ergab. Bariumneodecanoat ist in Isopar® E in der Kälte kaum löslich.

Nach weiteren 2 h war die Lösung gelbbraun. Eine gewichtsmäßig nicht erfaßbare Menge Bodensatz blieb am Boden des Reaktionsgefäßes, die Lösung blieb über 6 Wochen klar. Durch Abdestillieren des Lösungsmittels erhielten wir 138,7 g einer hochviskosen, rötlichbraunen Substanz;

9,57 g Lösungsmittel konnten also nicht entfernt werden.

Analysen: Ba = 19,6% (theor. 19,8%; für die Lösungsmittelfreie Substanz: 21,27%); Mg = 3,4% (theor. 3,5%, für die lösungsmittelfreie Substanz: 3,76%).

Die Substanz löste sich in Isopar® E wieder klar auf.

## Beispiel 2

Ein Ansatz wie in Beispiel 1 wurde mit der doppelten Menge $Bu_{1,5}Oc_{0,5}Mg$ umgesetzt.

Es ergab sich eine hochviskose gelbbraune Substanz in einer Ausbeute von 173,5 g, 11,12 g Lösungsmittel konnten nicht entfernt werden.

Die Substanz zeigte kein wesentlich geändertes Lösungsverhalten als die Substanz aus Beispiel 1.

Analysen: Ba = 15,6% (theor. 15,83%; für die lösungsmittelfreie Substanz 16,92%); Mg = 5,6% (theor. 5,60%; für die lösungsmittelfreie Substanz 5,99%).

## Beispiel 2a

Analog Beispiel 2 wurden umgesetzt:

0,1 Mol Bariumcapronat (36,73 g) und 1 Mol $Bu_{1,5}Oc_{0,5}Mg$ als 30%ige Lösung in einem Benzin des Siedebereichs 115 – 142° C. Die Lösung enthielt 4,38% Mg neben 0,1% Al.

Es ergab sich eine viskose gelbe Substanz in einer Ausbeute von 211 g. 8 g Lösungsmittel konnten nicht entfernt werden.

Analysen: Ba = 6,5% (theor. Wert; für die lösungsmittelfreie Substanz 6,76%); Mg = 11,5%.

## Beispiel 3

$$BaMg(C_6H_5)_2(O_2C-C_9H_{19})_2$$

0,05 Mol Bariumneodecanoat (23,97 g) wurden mit 0,05 Mol Diphenylmagnesiumlösung in Toluol (Mg-Gehalt: 1,36 Gew.-%, Al-Gehalt: 0,03 Gew.-%) 2 h auf Rückfluß erhitzt. Es löste sich alles zu einer gelbbraunen Lösung auf, die von Toluol befreit wurde. Rückstand: 34,30 g (theor. 32,88 g); 1,42 g Lösungsmittel konnten nicht entfernt werden.

Analysen: Ba = 19,74% (theor. 20,02%; ohne Lösungsmittel theor.: 20,88%); Mg = 3,49 (theor. 3,54%; ohne Lösungsmittel theor. 3,7%).

Die Substanz blieb nach Wiederauflösen mit dem abdestillierten Toluol in der Kälte klar gelöst.

## Beispiel 4

$$BaMgBusBu(O_2C-C_7H_{15})_2$$

0,05 Mol Barium-2-äthylhexanoat (21,17 g) wurden mit 0,05 Mol einer Butyl-s-Butylmagnesiumlösung (komplexiert mit 1/30 Mol Diäthyläther pro Mol Magnesium; 10 Gew.-%ige Lösung in Heptan) 3 h auf Rückfluß erhitzt. Bis auf einen geringen Bodensatz löste sich alles zu einer hellgelben Lösung, die auch beim Erkalten klar und dünnflüssig blieb. Das Lösungsmittel wurde abdestilliert und es verblieben 28,1 g Rückstand (theor. Ausbeute).

Analysen: Ba = 24,0% (theor. 24,45%); Mg = 4,3% (theor. 4,33%).

## Beispiel 5

$$BaMgBuMe(O_2C-C_9H_{19})(O_2C-C_7H_{15})$$

Wie in Beispiel 4 wurden umgesetzt:

0,025 Mol Bariumneodecanoat (11,98 g),
0,025 Mol Barium-2-äthylhexanoat (10,6 g) und
0,05 Mol Methylbutylmagnesium (unkomplexiert als 10gew.-%ige Lösung in Isopar® E). Ausbeute: 29,0 g (theor. 27,38 g).

Analysen: Ba = 22,9% (theor. 23,68%, lösungsmittelfreie Substanz theor. 25,08%); Mg = 4,0% (theor. 4,19%, lösungsmittelfreie Substanz theor. 4,44%).

**Patentansprüche**

1. Organische Barium-Magnesium-Komplexe der allgemeinen Formel

$$[Mg\,R^1_a R^2_b]_x\,[Ba(OOC-R^3)_2]_y \qquad (1)$$

in der $R^1$ und $R^2$ gleiche oder verschiedene geradkettige oder verzweigte Alkylreste oder Aryl- oder Aralkylrest bedeuten und in der $R^3$ ein Alkylrest mit mindestens 2 C-Atomen ist, wobei (a + b) gleich 2 ist und das Verhältnis x zu y 1 bis 4 beträgt.

2. Organische Barium-Magnesium-Komplexe gemäß Anspruch 1, dadurch gekennzeichnet, daß $R^3$ ein Alkylrest mit 5 bis 9 C-Atomen ist.

3. Organische Barium-Magnesium-Komplexe gemäß den Ansprüchen 1 und 2, dadurch gekennzeichnet, daß $R^1$ Butyl und $R^2$ Octyl ist.

4. Organische Barium-Magnesium-Komplexe gemäß den Ansprüchen 1 bis 3, dadurch gekennzeichnet, daß das Butyl-Octylverhältnis 3 : 1 beträgt.

5. Organische Barium-Magnesium-Komplexe gemäß den Ansprüchen 1 bis 4, dadurch gekennzeichnet, daß $R^3$ der Neononylrest ist.

6. $[Mg\,Bu_{1,5}Oc_{0,5}]_x\,[Ba(OOC-\text{neo-Nonyl})_2]_y$

7. Lösungen der Komplex-Verbindungen gemäß den Ansprüchen 1 bis 6 in Kohlenwasserstoff-Lösungsmitteln.

8. Verfahren zur Herstellung von in Kohlenwasserstoffen löslichen Barium-Magnesium-Komplexen gemäß den Ansprüchen 1 bis 6, dadurch gekennzeichnet, daß man Diorganomagnesium-Verbindungen der Formel

$$R^1_a R^2_b\,Mg \qquad (2)$$

mit Verbindungen der Formel

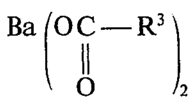

bei Temperaturen von 20 – 130°C, vorzugsweise bei 80 – 120°C, im Molverhältnis 1 : 1 bis 4 : 1 in Kohlenwasserstoffen umsetzt.

**Claims**

1. Organic-barium-magnesium complexes of the general formula

$$[Mg\,R^1_a R^2_b]_x[Ba(OOC-R^3)_2]_y \qquad (1)$$

in which $R^1$ and $R^2$ represent the same or different straight-chain or branched alkyl radicals or aryl radical or aralkyl radical, and in which $R^3$ represents an alkyl radical having at least 2 carbon atoms, (a + b) being equal to 2 and the proportion of x to y being from 1 to 4.

2. Organic barium-magnesium complexes according to claim 1, characterised in that $R^3$ represents an alkyl radical having from 5 to 9 carbon atoms.

3. Organic barium-magnesium complexes according to claims 1 and 2, characterised in that $R^1$ represents butyl and $R^2$ represents octyl.

4. Organic barium-magnesium complexes according to claim 1 to 3, characterised in that the ratio of butyl to octyl is 3 : 1.

5. Organic barium-magnesium complexes according to claims 1 to 4, characterised in that $R^3$ represents a neononyl radical.

6. $[Mg\,Bu_{1,5}Oc_{0,5}]_x\,[Ba(OOC-\text{neononyl})_2]_y$

7. Solutions of the complex compounds according to claims 1 to 6 in hydrocarbon solvents.

8. Process for the preparation of barium-magnesium complexes according to claims 1 to 6 that are soluble in hydrocarbons, characterised in that diorganomagnesium compounds of the formula

$$R^1_a R^2_b\,Mg \qquad (2)$$

are reacted with compounds of the formula

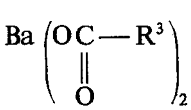

at temperatures of from 20 to 130° C, preferably at from 80 to 120° C, in a molar ratio of from 1 : 1 to 4 : 1 in hydrocarbons.

**Revendications**

1. Complexes organiques de baryum-magnésium qui répondent à la formule générale:

$$[Mg\,R^1_a R^2_b]_x [Ba(OOC-R^3)_2]_y \qquad (1)$$

dans laquelle $R^1$ et $R^2$ représentent chacun, indépendamment l'un de l'autre, un radical alkyle linéaire ou ramifié ou un radical aryle ou aralkyle, $R^3$ représente un radical alkyle contenant au moins deux atomes de carbone, la somme (a + b) est égale à 2 et le rapport x/y peut aller de 1 à 4.

2. Complexes organiques de baryum-magnésium selon la revendication 1, caractérisés en ce que $R_3$ représente un radical alkyle contenant de 5 à 9 atomes de carbone.

3. Complexes organiques de baryum-magnésium selon l'une des revendications 1 et 2, caractérisés en ce que $R^1$ représente un radical butyle et $R^2$ un radical octyle.

4. Complexes organiques de baryum-magnésium selon l'une quelconque des revendications 1 à 3, caractérisés en ce que le rapport butyle octyle est égal à 3 : 1.

5. Complexes organiques de baryum-magnésium selon l'une quelconque des revendications 1 à 4, caractérisésen ce que $R^3$ représente le radical néononyle.

6. $[Mg\,Bu_{1,5}Oc_{0,5}]_x\,[Ba(OOC-\text{néononyl})_2]_{-y}$

7. Solutions de complexes selon l'une quelconque des revendications 1 à 6 dans des solvants à bases d'hydrocarbures.

8. Procédé de préparation de complexes de baryum-magnésium solubles dans des hydrocarbures, selon l'une quelconque des revendications 1 à 6, procédé caractérisé en ce qu'on fait réagir des diorganomagnésiens de formule:

$$R^1_a R^2_b\,Mg \qquad (2)$$

avec des composés de formule:

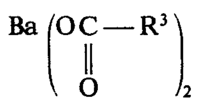

dans un rapport molaire de 1 : 1 à 4 : 1, à des températures de 20 à 130° C, de préférence de 80 à 120° C, dans des hydrocarbures.